(11) **EP 0 876 511 B1**

(12) **EUROPEAN PATENT SPECIFICATION**

(45) Date of publication and mention
of the grant of the patent:
**04.12.2002  Bulletin 2002/49**

(21) Application number: **97903104.4**

(22) Date of filing: **22.01.1997**

(51) Int Cl.[7]: **C12Q 1/68**

(86) International application number:
**PCT/US97/01179**

(87) International publication number:
**WO 97/027328 (31.07.1997 Gazette 1997/33)**

(54) **METHOD FOR ANALYZING REPEAT NUCLEIC ACID SEQUENCES**

VERFAHREN ZUR ANALYSE VON NUKLEINSÄURE-WIEDERHOLUNGSSEQUENZEN

METHODE D'ANALYSE DE SEQUENCES REPETEES D'ACIDE NUCLEIQUE

(84) Designated Contracting States:
**AT BE CH DE ES FR GB IT LI NL**

(30) Priority: **26.01.1996  US 591883**

(43) Date of publication of application:
**11.11.1998  Bulletin 1998/46**

(73) Proprietor: **Abbott Laboratories
Abbott Park, Illinois 60064-3500 (US)**

(72) Inventor: **GORDON, Julian
Lake Bluff, IL 60044 (US)**

(74) Representative: **Modiano, Guido, Dr.-Ing. et al
Modiano, Josif, Pisanty & Staub,
Baaderstrasse 3
80469 München (DE)**

(56) References cited:
**WO-A-94/28175          WO-A-95/25179
WO-A-95/25815          WO-A-95/30774
WO-A-96/09532          WO-A-96/36731**

• **NUCLEIC ACIDS RESEARCH, vol. 22, no. 9, -
1994 pages 1701-1704, XP002033815 WEHNERT
M. S. ET AL.,: "A rapid scanning strip for tri- and
dinucleotide short tandem repeats"**
• **HUMAN MOLECULAR GENETICS, vol. 3, no. 4, -
1994 XP002033816 ARMOUR J.A.L. ET AL.,:
"Isolation of human simple repeat loci by
hybridization selection"**

SEQUENCE LISTING

(1) GENERAL INFORMATION:

   (i) APPLICANT: J. Gordon

   (ii) TITLE OF INVENTION: METHOD FOR ANALYZING REPEAT
      NUCLEIC ACID SEQUENCES

   (iii) NUMBER OF SEQUENCES: 18

   (iv) CORRESPONDENCE ADDRESS:

      (A) ADDRESSEE: Abbott Laboratories
      (B) STREET: 100 Abbott Park Road
      (C) CITY: Abbott Park
      (D) STATE: Illinois
      (E) COUNTRY: USA
      (F) ZIP: 60064-3500

   (v) COMPUTER READABLE FORM:

      (A) MEDIUM TYPE: Floppy disk
      (B) COMPUTER: Macintosh
      (C) OPERATING SYSTEM: System 7.0.1
      (D) SOFTWARE: Microsoft Word 5.1a

(vi) CURRENT APPLICATION DATA:

    (A) APPLICATION NUMBER:
    (B) FILING DATE:
    (C) CLASSIFICATION:

(viii) ATTORNEY/AGENT INFORMATION:

    (A) NAME: Paul D. Yasger
    (B) REGISTRATION NUMBER: 37,477
    (C) DOCKET NUMBER: 5859.US.01

(ix) TELECOMMUNICATION INFORMATION:

    (A) TELEPHONE: 708/938-3508
    (B) TELEFAX: 708/938-2623
    (C) TELEX:

(2) INFORMATION FOR SEQ ID NO:1:

    (i) SEQUENCE CHARACTERISTICS:

        (A) LENGTH: 30 base pairs
        (B) TYPE: nucleic acid
        (C) STRANDEDNESS: single
        (D) TOPOLOGY: linear

    (ii) MOLECULE TYPE: synthetic DNA

    (xi) SEQUENCE DESCRIPTION: SEQ ID NO:1:

```
GACGACGACG ACGACGACGA CGACGACGAC                                    30
```

(2) INFORMATION FOR SEQ ID NO:2:

    (i) SEQUENCE CHARACTERISTICS:

        (A) LENGTH: 60 base pairs
        (B) TYPE: nucleic acid
        (C) STRANDEDNESS: single
        (D) TOPOLOGY: linear

    (ii) MOLECULE TYPE: synthetic DNA

    (xi) SEQUENCE DESCRIPTION: SEQ ID NO:2:

```
GACGACGACG ACGACGACGA CGACGACGAC GACGACGACG ACGACGACGA          50

CGACGACGAC                                                      60
```

**EP 0 876 511 B1**

(2) INFORMATION FOR SEQ ID NO:3:

(i) SEQUENCE CHARACTERISTICS:

(A) LENGTH: 90 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:3:

```
GACGACGACG ACGACGACGA CGACGACGAC GACGACGACG ACGACGACGA    50

CGACGACGAC GACGACGACG ACGACGACGA CGACGACGAC                90
```

(2) INFORMATION FOR SEQ ID NO: 4:

(i) SEQUENCE CHARACTERISTICS:

(A) LENGTH: 120
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO: 4:

```
GACGACGACG ACGACGACGA CGACGACGAC GACGACGACG ACGACGACGA    50

CGACGACGAC GACGACGACG ACGACGACGA CGACGACGAC GACGACGACG    100

ACGACGACGA CGACGACGAC                                      120
```

(2) INFORMATION FOR SEQ ID NO:5:

(i) SEQUENCE CHARACTERISTICS:

(A) LENGTH: 150 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:5:

```
GACGACGACG ACGACGACGA CGACGACGAC GACGACGACG ACGACGACGA      50

CGACGACGAC GACGACGACG ACGACGACGA CGACGACGAC GACGACGACG     100

ACGACGACGA CGACGACGAC GACGACGACG ACGACGACGA CGACGACGAC     150
```

(2) INFORMATION FOR SEQ ID NO:6:

    (i) SEQUENCE CHARACTERISTICS:

        (A) LENGTH: 60 base pairs
        (B) TYPE: nucleic acid
        (C) STRANDEDNESS: single
        (D) TOPOLOGY: linear

    (ii) MOLECULE TYPE: synthetic DNA

    (xi) SEQUENCE DESCRIPTION: SEQ ID NO:6:

```
GCCGCCGCCG CCGCCGCCGC CGCCGCCGCC GCCGCCGCCG CCGCCGCCGC      50

CGCCGCCGCC                                                 60
```

(2) INFORMATION FOR SEQ ID NO:7:

    (i) SEQUENCE CHARACTERISTICS:

        (A) LENGTH: 120 base pairs
        (B) TYPE: nucleic acid
        (C) STRANDEDNESS: single
        (D) TOPOLOGY: linear

    (ii) MOLECULE TYPE: synthetic DNA

    (xi) SEQUENCE DESCRIPTION: SEQ ID NO:7:

```
GCCGCCGCCG CCGCCGCCGC CGCCGCCGCC GCCGCCGCCG CCGCCGCCGC      50

CGCCGCCGCC GCCGCCGCCG CCGCCGCCGC CGCCGCCGCC GCCGCCGCCG     100

CCGCCGCCGC CGCCGCCGCC                                      120
```

(2) INFORMATION FOR SEQ ID NO:8:

    (i) SEQUENCE CHARACTERISTICS:

        (A) LENGTH: 180 base pairs

(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:8:

```
GCCGCCGCCG  CCGCCGCCGC  CGCCGCCGCC  GCCGCCGCCG  CCGCCGCCGC   50

CGCCGCCGCC  GCCGCCGCCG  CCGCCGCCGC  CGCCGCCGCC  GCCGCCGCCG   100

CCGCCGCCGC  CGCCGCCGCC  GCCGCCGCCG  CCGCCGCCGC  CGCCGCCGCC   150

GCCGCCGCCG  CCGCCGCCGC  CGCCGCCGCC                          180
```

(2) INFORMATION FOR SEQ ID NO:9:

(i) SEQUENCE CHARACTERISTICS:

(A) LENGTH: 240 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:9:

```
GCCGCCGCCG  CCGCCGCCGC  CGCCGCCGCC  GCCGCCGCCG  CCGCCGCCGC   50

CGCCGCCGCC  GCCGCCGCCG  CCGCCGCCGC  CGCCGCCGCC  GCCGCCGCCG   100

CCGCCGCCGC  CGCCGCCGCC  GCCGCCGCCG  CCGCCGCCGC  CGCCGCCGCC   150


GCCGCCGCCG  CCGCCGCCGC  CGCCGCCGCC  GCCGCCGCCG  CCGCCGCCGC   200

CGCCGCCGCC  GCCGCCGCCG  CCGCCGCCGC  CGCCGCCGCC               240
```

(2) INFORMATION FOR SEQ ID NO:10:

(i) SEQUENCE CHARACTERISTICS:

(A) LENGTH: 150 base pairs
(B) TYPE: nucleic acid
(C) STRANDEDNESS: double
(D) TOPOLOGY: linear

(ii) MOLECULE TYPE: genomic DNA

(xi) SEQUENCE DESCRIPTION: SEQ ID NO:10:

```
CTGCTGCTGC TGCTGCTGCT GCTGCTGCTG CTGCTGCTGC TGCTGCTGCT     50

GCTGCTGCTG CTGCTGCTGC TGCTGCTGCT GCTGCTGCTG CTGCTGCTGC    100

TGCTGCTGCT GCTGCTGCTG CTGCTGCTGC TGCTGCTGCT GCTGCTGCTG    150
```

(2) INFORMATION FOR SEQ ID NO:11:

(i) SEQUENCE CHARACTERISTICS:

    (A) LENGTH: 14 base pairs
    (B) TYPE: nucleic acid
    (C) STRANDEDNESS: single
    (D) TOPOLOGY: linear

(ii) MOLECULE TYPE: synthetic DNA

(ix) FEATURE:

    (A) NAME/KEY: 5' biotin
    (B) LOCATION: 1

(xi) SEQUENCE DESCRIPTION: SEQ ID NO:11:

```
CACCACCAGC AACA                                            14
```

(2) INFORMATION FOR SEQ ID NO:12:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: synthetic DNA

(ix) FEATURE:

    (A) NAME/KEY: 5' biotin
    (B) LOCATION: 1

(xi) SEQUENCE DESCRIPTION: SEQ ID NO:12:

```
GGCCCAGAGT TTCCGTGATG                                      20
```

(2) INFORMATION FOR SEQ ID NO:13:

(i) SEQUENCE CHARACTERISTICS:

(A) LENGTH: 30 base pairs
(B) TYPE: nucleic acid
(C) STRANDEDNESS: double
(D) TOPOLOGY: linear

(ii) MOLECULE TYPE: genomic DNA

(xi) SEQUENCE DESCRIPTION: SEQ ID NO:13:

CGGCGGCGGC GGCGGCGGCG GCGGCGGCGG CGGCGGCGGC GGCGGCGGCG    50

GCGGCGGCGG CGGCGGCGGC GGCGGCGGCG GCGGCGGCGG CGGCGGCGGC    100
GGCGGCGGCG GCGGCGGCGG CGGCGGCGGC GGCGGCGGCG GCGGCGGCGG    150
CGGCGGCGGC GGCGGCGGCG GCGGCGGCGG CGGCGGCGGC GGCGGCGGCG    200
GCGGCGGCGG CGGCGGCGGC GGCGGCGGCG GCGGCGGCGG              240

(2) INFORMATION FOR SEQ ID NO:14:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: synthetic DNA

(ix) FEATURE:

(A) NAME/KEY: 5' biotin
(B) LOCATION: 1

(xi) SEQUENCE DESCRIPTION: SEQ ID NO:14:

ACCTCTGCAG AAATGGGCGT TCTG3CCCTC                          30

(2) INFORMATION FOR SEQ ID NO:15:

(i) SEQUENCE CHARACTERISTICS:

        (A) LENGTH: 38 base pairs
        (B) TYPE: nucleic acid
        (C) STRANDEDNESS: single
        (D) TOPOLOGY: linear

    (ii) MOLECULE TYPE: synthetic DNA

    (ix) FEATURE:

        (A) NAME/KEY: 5' biotin
        (B) LOCATION: 1

    (xi) SEQUENCE DESCRIPTION: SEQ ID NO:15:


        CGGAATTCGC TAGCGCCGGG AGCCCGCCCC CGAGAGGT                        38


    (2) INFORMATION FOR SEQ ID NO:16:

            (i) SEQUENCE CHARACTERISTICS:

            (A) LENGTH: 15 base pairs
            (B) TYPE: nucleic acid
            (C) STRANDEDNESS: single
            (D) TOPOLOGY: linear

    (ii) MOLECULE TYPE: synthetic DNA

    (xi) SEQUENCE DESCRIPTION: SEQ ID NO:16:


        GACGACGACG ACGAC                                                 15


    (2) INFORMATION FOR SEQ ID NO:17:

            (i) SEQUENCE CHARACTERISTICS:

            (A) LENGTH: 156 base pairs
            (B) TYPE: nucleic acid
            (C) STRANDEDNESS: single
            (D) TOPOLOGY: linear

    (ii) MOLECULE TYPE: synthetic DNA

    (xi) SEQUENCE DESCRIPTION: SEQ ID NO:17:


        GACGACGACG ACGACGACGA CGACGACGAC GACGACGACG ACGACGACGA     50

```
CGACGACGAC GACGACGACG ACGACGACGA CGACGACGAC GACGACGACG    100

ACGACGACGA CGACGACGAC GACGACGACG ACGACGACGA CGACGACGAC    150

GACGAC                                                    156
```

(2) INFORMATION FOR SEQ ID NO:18:

(i) SEQUENCE CHARACTERISTICS:

(A) LENGTH: 300 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:18:

```
GACGACGACG ACGACGACGA CGACGACGAC GACGACGACG ACGACGACGA    50

CGACGACGAC GACGACGACG ACGACGACGA CGACGACGAC GACGACGACG    100

ACGACGACGA CGACGACGAC GACGACGACG ACGACGACGA CGACGACGAC    150

GACGACGACG ACGACGACGA CGACGACGAC GACGACGACG ACGACGACGA    200

CGACGACGAC GACGACGACG ACGACGACGA CGACGACGAC GACGACGACG    250

ACGACGACGA CGACGACGAC GACGACGACG ACGACGACGA CGACGACGAC    300
```

**Description**

Field of the Invention

The present invention relates to sequentially repeated nucleic acid sequences, and in particular, relates to methods for analyzing the number of times a repeated sequence is sequentially repeated.

Background of the Invention

Certain regions of the human genome contain sequentially repeated nucleic acid sequences, variously referred to as tandem repeats. A repeated sequence of three nucleotides is commonly referred to as a "triplet repeat" and although a certain multiple of a repeated sequence may be normal, genetic mutations can increase the multiple of the repeat causing an unstable structure where the multiple tends to increase generation by generation. Unfortunately, when the multiple is increased above a certain number, the repeated region can be responsible for diseases having severe outward manifestations such as mental retardation, neuromuscular degenerative disorders, ataxia, involuntary movement, epilepsy, dementia and nerve palsy.

For example, healthy individuals carry a CGG triplet repeat where the CGG sequence is repeated less than 50 times. If this length is exceeded, instability results. When the number of repeats exceeds 200, a genetic disorder called Fragile X Syndrome occurs. The unstable length range (i.e. CGG repeat multiples between 50 and 200) is referred to as a "pre-mutation" and results in a genetic phenomenon referred to above where future generations are susceptible to receiving a fully mutated sequence. A similar genetic disorder called Myotonic Dystrophy is characterized by persons having the sequence CTG repeated more than about 100 times. Individuals in which CTG repeats in the range of about 52 and about 90 times are in the pre-mutation stage, and individuals in which CTG repeats between about 5 and about 37 times are normal. Dentatorubral-Pallidoluysian Atrophy is a further example of a genetic disorder characterized by triplet repeat expansion.

Other diseases, such as those attributable to, for example, a virus, can be characterized by the presence of a unique nucleic acid sequence in a genome, and detection of that particular target sequence is relatively straight forward. However, detecting genetic disorders characterized by expanded repeat sequences is difficult because the sequences are redundant, and it is the extent of this redundancy, not the presence of the sequence, per se, which is indicative of the disease. Thus, for example, a solid support material that is coated with a sequence which is complementary to a repeat sequence will hybridize with the repeat sequence regardless of the length of the sequence. Hence, while it is possible to detect the presence of the repeat sequence, determining the length of the sequence remains a problem. As a result, conventional capture reagents generally are not employed to detect genetic disorders which arise when a repeated sequence exceeds a certain multiple.

Thus, in current research practice, tandem repeat sequences are analyzed by gel electrophoresis, typically using gel systems employed for sequencing reactions. In clinical practice, because of the difficulty in resolving size differences of repeat sequences, restriction fragmentation and Southern blotting are utilized.

Summary of the Invention

The present invention provides a method for analyzing a target sequence to determine the number of times a repeated sequence is found in the target sequence relative to a set of oligonucleotides which have different multiples of a sequence complementary to the repeated sequence. The method is rapid and easily automated and therefore requires minimal technician interaction. A repeated sequence conforms to the formula $A_pC_yG_aT_z$ wherein: T can be substituted with U; A, C, G, T and U represent bases; p, y, a, and z are between 0 and 5; the sum of p+y+a+z is at least 2; and A, C, G, and T or U can be in any order. The repeated sequence is tandemly repeated at least twice in a target sequence and the method for analyzing such a target sequence according to the present invention, comprises contacting a test sample with a target sequence capture reagent to form target sequence/capture oligonucleotide hybrids. The target sequence capture reagent comprises at least two capture oligonucleotides immobilized to a solid support wherein the capture oligonucleotides have different multiples of the sequence complementary to the repeated sequence. Increasing the stringency in the environment of any hybrids formed on the target sequence capture reagent causes the hybrids to progressively dissociate. A progressive hybrid dissociation pattern can be detected to determine the multiple of the repeated sequence in the target sequence, wherein this multiple is determined relative to the multiples of complementary sequences found in the capture oligonucleotides.

Also provided is a target sequence capture reagent for capturing a target sequence comprising a repeated sequence having the formula described above. The capture reagent comprises at least two oligonucleotides, having different multiples of a sequence complementary to the repeated sequence, immobilized to a solid support material.

Brief Description of the Drawings

Figure 1 schematically shows a target sequence capture reagent.

Figures 2 and 3a-3c schematically illustrate an optical waveguide device and the concept behind use of an optical waveguide device to detect hybrid formation or dissociation.

Figures 4 and 5 are schematic illustrations of target sequence capture reagents.

Figures 6 and 8 are graphic representations of computer generated hybrid melting temperatures as a function of the number of repeated sequences.

Figures 7a-7e, 9a-9e, and 10(a)-10(d) schematically demonstrate the analysis of a target sequence according to the present invention.

Detailed Description of the Invention

As used herein, the term "repeated sequence" means a base or nucleic acid sequence that is sequentially or tandemly repeated. Also, for purposes of the present invention, a "target sequence" means a nucleic acid sequence comprising a repeated sequence, and the region of the target sequence containing the repeated sequence will be called the "repeat region". For example, in a target sequence comprising the series of bases CTGCTGCTG, a repeated sequence is CTG. "Base" as used herein has its ordinary meaning and includes modified and naturally occurring deoxyribonucleotides and ribonucleotides such as adenine (A), cytosine (C), guanine (G), thymine (T), and uracil (U). A repeated sequence can be represented by the formula $A_p C_y G_a T_z$ where T can be substituted with U; A, C, G, T and U represent bases; p, y, a, and z represent the number of times each base occurs in a series of bases that is repeated and can range between 0 and 5; the sum of p+y+a+z is typically 2 or more and preferably 3; A, C, G, and T or U can be in any order in the repeated sequence; and the repeated sequence is typically repeated in a target sequence 2 or more times, more typically between 5 and 200 times. As used in characterizing the repeated sequence, the phrase "any order" is intended to mean that bases in the repeated sequence can be in any sequence, and that multiple occurrences of the same base can be next to one another in adjacent series, or separated by at least one different intervening base. For example, in the case where p is equal to 2; y is equal to 1; a and z are equal to 0; and the repeated sequence is repeated twice, the following sequences are possible: AACAAC; ACAACA; or CAACAA.

Complementary double-stranded nucleic acid sequences, including complementary sequences having repeat regions, are known to dissociate under various stringency conditions. Generally, hybridized nucleic acid sequences or "hybrids" having varying lengths also have varying hybridization strengths with longer sequences forming stronger hybrids than shorter sequences. Thus, more stringent conditions are required to dissociate longer hybrids. The method of the present invention exploits the stringency differences that are required to dissociate different length hybrids to indicate the multiple of a repeated sequence in a target sequence.

The invention is particularly suited for detecting genetic disorders, or susceptibility thereto, by determining a repeated sequence's multiple in a target sequence relative to the multiple of its complement in a capture oligonucleotide. Table 1 provides a list of exemplary genetic diseases characterized by repeated sequence expansion, the repeated sequence, and the range of multiples of the repeated sequence which characterizes normal individuals, pre-mutation individuals and individuals having the disorder.

Table 1

| Disease | Rpt'd Sequence | Normal | Pre-Mutation | Full Mutation |
|---|---|---|---|---|
| Fragile X | CGG | 6-54 | 52-199 | 200+ |
| Myotonic Dystrophy | CTG | 5-37 | 52-90 | 100+ |
| Huntington's Disease | CAG | 30-34 | 34-41 | 42+ |
| Spino-bulbar Muscular Atrophy | CAG | 11-30 | 31-46 | 47+ |
| Spino-cerebellar Ataxia | CAG | 25-36 | 36-42 | 43+ |
| Machado-Joseph Disease | CAG | 13-36 | 39-68 | 69+ |

By exploiting the stringency differences required to dissociate variable length hybrids comprising repeated sequences, such genetic abnormalities can be detected according to the present invention. For example, a test sample, and any target sequences contained therein, can be contacted with a target sequence capture reagent comprising at least two capture oligonucleotides having various multiples of the repeated sequence's complementary sequence. Target sequence/capture oligonucleotide hybrids are thereby formed and subject to incremental increases in stringency. Incremental stringency increases cause the hybrids to progressively dissociate based upon the amount of hybridization between complementary sequences, with the hybrids formed through the greatest amount of base pairing dissociating last. Detecting the resulting progressive hybrid dissociation pattern enables a determination of the number of times the repeated sequences is found in the target sequence, relative to the number of its complementary sequence in the capture oligonucleotides.

A "test sample" refers to a material suspected of containing a target sequence. The test sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid and the like, or fermentation broths, cell cultures, chemical reaction mixtures and the like. The test sample can be used (i) directly as obtained from the source or (ii) following a pre-treatment to modify the character of the sample. Thus, the test sample can be pretreated prior to use by, for example, preparing plasma from blood, preparing liquids from solid materials, diluting viscous fluids, filtering liquids, distilling liquids, concentrating liquids, inactivating interfering components, adding reagents, and the like. Test samples also can be pretreated to digest, restrict or render double stranded nucleic acid sequences single stranded. Moreover, test samples may be pretreated to accumulate, purify, amplify or otherwise concentrate target sequences that may be contained therein.

As mentioned above, target sequences may be amplified and amplification reactions well known in the art such as, for example the polymerase chain reaction (PCR), which has been described in U.S. Patents 4,683,195 and 4,683,202, can be employed for this purpose. Suitable amplification primers can be selected based upon sequences which are known (or can be determined) to flank the region containing the repeat sequence. It should be noted that repeated sequences can be rich in GC content and amplifying such regions using PCR may produce amplification products with secondary structure. Methods for avoiding secondary structure during amplification in GC rich regions include using a 7-deaza-2'-deoxyguanosine-5'-triphoshpate, a modified G nucleotide described in International Patent Application No. WO 90/03443. Accordingly, when PCR is used to amplify a GC rich repeat sequence it is preferred to employ 7-deaza-2'-deoxyguanosine-5'-triphoshpate. Also, Schalling, M., *et. al.*, *Nature Genetics,* vol. 4, pp. 135-139 (1993) describe a preferred and modified-LCR procedure adapted to amplify a target sequence more efficiently through multiple ligations of multiple probes that hybridize with the repeat region of a target sequence.

Whether or not pretreated, the test sample is contacted with capture oligonucleotides and the term "capture oligonucleotides", which is intended to encompass polynucleotides as well, may comprise, for example, nucleic acid, ribonucleic acid and nucleic acid analogs such as uncharged nucleic acid analogs including but not limited to peptide nucleic acids (PNAs) which are disclosed in International Patent Application WO 92/20702 or morpholino analogs which are described in U.S. Patents Numbered 5,185,444, 5,034,506, and 5,142,047.

Capture oligonucleotides are at least partially complementary to the repeat region of a target sequence and therefore have at least one sequence of bases that is complementary to the repeated sequence. However, the number of times this complementary sequence is repeated in any particular capture oligonucleotide can be more, the same or less than the number of times the repeated sequence is repeated in the target sequence. Thus, the capture oligonucleotides have a sequence of bases that is determined by the repeated sequence, but because capture oligonucleotides need only be partially complementary to the target sequence, any particular capture oligonucleotide can be longer, shorter or the same length as the target sequences. Hence, using the repeated sequence example presented above where p is equal to 2, y is equal to 1, a and z are equal to 0, the repeated sequence was repeated twice and the order of the repeated sequence was AAC; a capture oligonucleotide designed to hybridize with this sequence could have the following sequences: TTG, TTGTTG or TTGTTGTTG.

While a portion of a capture oligonucleotide sequence is complementary to the repeat region of a target sequence, the capture oligonucleotides generally are not perfectly complementary to the target sequences. This is the case for several reasons. First, as previously mentioned, capture oligonucleotides can have varying multiples of the sequence that is complementary to the repeated sequence. For example, one of the capture oligonucleotides on the target sequence capture reagent may consist of a sequence which is the complement of the repeated sequence in the exact multiple in which the repeated sequence is actually found in the target sequence--hence rendering that capture oligonucleotide exactly complementary to the target sequence's repeat region. On the other hand, other capture oligonucleotides forming the target sequence capture reagent may contain the complement of the repeated sequence in multiples greater or lesser than the actual multiple of the repeated sequence present in the target sequence. Additionally, even if the multiple of the repeated sequence in the target sequence matches the number of repeats in a capture oligonucleotide, the capture oligonucleotide may comprise additional base pairs, such as nucleic acid tails which can be employed, for example, to immobilize the capture oligonucleotide to a solid support material, as will be explained in more detail below. Alternatively, a test sample may contain amplified target sequences obtained, for example, by using amplification primers or probes that hybridize with regions adjacent to (or flank) a repeated region. Therefore, the resultant amplification product may comprise sequences in addition to the repeated sequence. Moreover, in cases where a test sample is pretreated by for example, digestion or restriction, the target sequence may also comprise regions adjacent to the repeated region. Hence, the sequence of the a capture oligonucleotide is not always the perfect complement of the repeat region.

As previously mentioned, capture oligonucleotides can be immobilized to solid support materials to form a target sequence capture reagent. As used herein a "solid support material" means any of the materials well known for this purpose which are insoluble, or can be made insoluble by a subsequent reaction. Solid support materials thus can be, for example, latex, plastic, derivatized plastic, magnetic or nonmagnetic metal, glass or silicon surface or surfaces of test tubes, microtiter wells, sheets, chips, and other configurations known to those of ordinary skill in the art. It is contemplated and within the scope of the invention that solid support materials may also comprise any suitable porous material with sufficient porosity to allow, when necessary, access by target sequences.

Capture oligonucleotides may be bound to a support material using any of the well known methodologies such as, for example, adsorption, covalent linkages, gold thiolate interactions or, as mentioned previously, polynucleotide tails.

While capture oligonucleotides may be randomly immobilized to a support material, preferably, capture oligonucleotides are immobilized to a solid support material in an "array" which means that a pattern of at least two discreet oligonucleotide capture sites is formed on the support material. Individual capture sites comprise capture oligonucleotides having the same multiple of the sequence complementary to the repeated sequence. Hence, one site may comprise a capture oligonucleotide having the repeated sequence's complement repeated five times and another capture site may comprise a capture oligonucleotide having the repeated sequence's complement repeated ten times. Arrays of capture oligonucleotide sites permit distinctions to be made with respect to whether or not a repeat sequence is hybridized to a particular site. Typically, arrays of capture oligonucleotides are formed by physically spacing the capture oligonucleotide sites from one another. As a result, capture oligonucleotides comprising the same multiple of a given sequence are segregated from capture oligonucleotides having a different multiple of the sequence.

The distance between capture oligonucleotides in an array is largely a matter of choice for one skilled in the art based upon a selected detection system's resolution capabilities. Similarly, the size of the sites where the oligonucleotides are immobilized, as well as the concentration of oligonucleotides immobilized at these sites, is a matter of choice for one skilled in the art which also can be based upon the detection system employed. Specifically, sensitive detection systems can detect whether or not repeat sequences are hybridized to capture reagents having high surface densities of tightly spaced capture oligonucleotides. Thus, if a CCD camera were employed to observe the presence or absence of hybrids at a particular site, smaller sites, smaller oligonucleotide concentrations, and smaller distances between oligonucleotides could be employed than when a simple mechanically-unassisted observation of an array is made.

Arrays of capture oligonucleotides may include simple patterns such as, for example, at least two capture oligonucleotide spots on a support material wherein the capture oligonucleotide spots comprise different multiples of the sequence which is complementary to the repeat sequence. More complex patterns can be made using methodologies described in U.S. Patent No. 5,405,783, U.S. Patent No. 5,412,087, Southern E.M., *et. al.*, Nucleic Acids Research, Vol. 22, No. 8, pp. 1368-1373 (1994) and Maskos U., *et. al.*, Nucleic Acids Research, Vol. 21, No. 20, pp. 4663-4669 (1993).

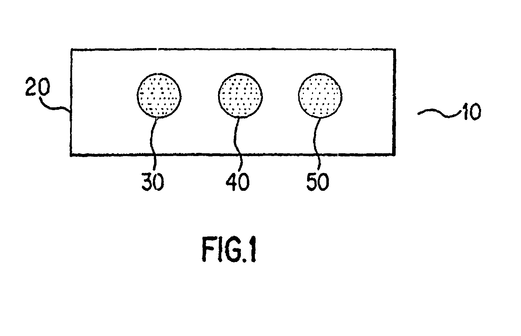

A preferred pattern of oligonucleotides is shown in Figure 1 which illustrates repeat capture reagent 10. As shown by Figure 1, a solid support 20 has capture oligonucleotides immobilized thereon to form capture sites 30, 40, and 50. According to the present invention, the capture site 30 may have capture oligonucleotides having 4 multiples of a repeat sequence's complement; capture site 40 may have capture oligonucleotides having 7 multiples of a repeated sequence's complement; and capture site 50 may have capture oligonucleotides having 10 multiples of a repeated sequence's complement.

Contact between the complementary regions of the target sequence's repeat region and the repeat capture reagent's capture oligonucleotides results in the formation of target sequence/capture oligonucleotide hybrids having various degrees of hybridization strength because the capture oligonucleotides are complementary to the repeat region of the target sequence in various degrees. Additionally, the degree of hybridization (or amount of base pairing) between a target sequence's repeat region and any particular capture oligonucleotide generally will be to the greatest extent possible because hybrids having more base pairing are more stable than hybrids having less base pairing. Thus, hybrids formed through base pairing to the greatest extent possible are favored hybrids and will be the predominant target sequence/capture oligonucleotide hybrid. However, as stated previously, the sequences having the greatest degree of hybridization will be the most stable and therefore, the most difficult to dissociate.

Incremental stringency increases in the environment of the hybrids causes them to progressively dissociate. As used herein "progressively dissociate" means dissociation based upon the amount of base pairing interaction which occurs between complementary single stranded sequences. Thus, progressive dissociation occurs according to the present invention as incremental stringency increases are caused in the environment surrounding target sequence/capture oligonucleotide sequence hybrids. Based upon the amount of base pairing, incremental stringency increases yield a staged dissociation pattern which can be detected to indicate the multiple of a repeated sequence in the target sequence. Specifically, in response to increasing stringency conditions, the weakest hybrids, or those formed with the least amount of base pairing, will dissociate first and those formed through the greatest amount of base pairing will dissociate last. Thus, by detecting dissociation of the various target sequence/capture oligonucleotide hybrids, the multiple of the repeated sequence can be determined relative to the capture oligonucleotides. It will be understood, of course, that a progressive dissociation may include a dissociation patterns that include a single dissociation event where all hybrids dissociate at once or dissociation patterns where multiple dissociation events take place at different stringencies.

According to the present invention, the multiple of a repeated sequence relative to its complement in the capture oligonucleotides can be determined. As used herein, "relative to the capture oligonucleotides" means that an assessment of the number of times a repeated sequence occurs in a target sequence can be made with respect to the number of times its complement is found in the capture oligonucleotides. Accordingly, a characterization of the repeated sequence multiple in a target sequence, as it relates to a particular capture oligonucleotide, may be made. The multiple of the repeated sequence in a particular target sequence relative to a capture oligonucleotide may include characterizations such as, for example, "less than", "greater than", or "less than or equal to" the multiple of the repeated sequences complement in a capture oligonucleotide.

For example, hybridization between a solution of repeat sequences and a repeat capture reagent comprising three capture oligonucleotides each having a different multiple of the sequence complementary to the repeated sequence could include situations where (i) the multiple of the repeated sequence in the target sequence is less than the multiple of its complement found in all of the capture oligonucleotides, (ii) the multiple of the repeated sequence in the target sequences is the same as the multiple of its complement found in a mid-sized capture oligonucleotide or (iii) the multiple of the repeated sequence in the target sequence is greater than the multiple of its compliment found in any of the capture oligonucleotides. For purposes of discussion with respect to a repeat capture reagent, capture oligonucleotides having the smallest multiple of a repeated sequences complement will be called the shortest capture oligonucleotide, capture oligonucleotides having the greatest multiple of a repeated sequences complement will be called the longest capture oligonucleotide, and capture oligonucleotides having multiples of a repeated sequence's complement between those of the shortest and longest capture oligonucleotides will be called mid-sized capture oligonucleotides.

With respect to the previously mentioned three possible scenarios, in the first instance, complete base pairing between complementary portions of the target sequences and all capture oligonucleotides would occur and therefore, all resulting hybrids would have the same hybridization strength. Hence, all of the target would dissociate from the capture oligonucleotides at a common stringency. Upon detection of this progressive dissociation pattern, it could be determined that the target sequences contained a repeated sequence multiple less than or equal to the multiple of repeated sequence complement in the shortest capture oligonucleotide.

In the second instance, incomplete base pairing would occur between complementary portions of the target sequences and the shortest capture oligonucleotides, and complete base pairing could occur between complementary portions of the target sequences and the mid-sized and longest capture oligonucleotides. Hence, hybrids formed with the shortest capture oligonucleotides would dissociate first and the others would dissociate at a greater common stringency. Upon detecting this progressive dissociation, it could be determined that the repeated sequence multiple was greater than the multiple of its complement in the shortest capture oligonucleotide, greater than or equal to the multiple of its complement in the mid-sized capture oligonucleotide, and less than the multiple of its complement in the longest capture oligonucleotide.

In the third case, hybrids would again form with all capture oligonucleotides, but the target sequences would overhang all of the capture oligonucleotides because the multiple of the repeated sequence is greater than the multiple of its complement found in any of the capture oligonucleotides. Nevertheless, the longest capture oligonucleotide would form the strongest hybrid because it would have the greatest amount of base pairing with the repeat region of the target sequences. Therefore, under incrementally increasing stringency conditions, hybrids will dissociate first from the shortest capture oligonucleotide; followed by dissociation of hybrids formed between the target sequences and the midsized capture oligonucleotides; which in turn would be followed by dissociation of hybrids formed between the repeat sequences and the longest capture oligonucleotides. Detecting this progressive dissociation would indicate that the multiple of the repeated sequence was greater than or equal to the multiple of its complement found in the longest capture oligonucleotide.

Incremental increases in stringency may be brought about by any chemical or physical phenomenon which dissociates hybrid sequences and has an effectiveness which is based upon the amount of base pairing between complementary nucleic acid sequences. For example, pH changes and ionic strength changes dissociate hybrid sequences in various degrees based upon the amount of base pairing. Specifically, lower pHs and ionic strengths are required to dissociate longer hybrids than are required to dissociate shorter hybrids. Similarly, a higher melt temperature (Tm) is required to dissociate longer sequences from one another than is required to dissociate shorter sequences from one another. Thus, temperature, pH and ionic strength changes can be employed as means for changing the stringency of the environment surrounding hybrids.

For example, when pH or ionic strength is employed to increase stringency and dissociate hybrids, buffers having varying pH's or ionic strengths can be sequentially contacted with the hybrids to thereby incrementally increase stringency and cause a progressive hybrid dissociation. Alternatively, for example, when temperature is employed to increase stringency, the temperature in the environment of the hybrids can be incrementally increased to thereby increase stringency and cause dissociation in a progressive manner. Increasing stringency is preferably achieved by continuous temperature elevation, as this potentially eliminates the need for multiple fluid transfers.

"The environment of the hybrids" is the area surrounding the hybrids in which incremental stringency increases cause dissociation in the manner discussed above.

Detecting hybrid dissociation can be assisted by incorporating detectable labels into the target sequences, the capture oligonucleotides, or both the repeat sequences and the capture oligonucleotides. The term label refers to any moiety having a property or characteristic which is capable of detection. A label can be directly detectable, as with, for example, radioisotopes, fluorophores, chemiluminophores, enzymes, colloidal particles, fluorescent microparticles, intercalation dyes and the like; or a label may be indirectly detectable, as with, for example, specific binding members. It will be understood that direct labels may require additional components such as, for example, substrates, triggering reagents, light, and the like to enable detection of the label.

When indirect labels are used, they are typically used in combination with a "conjugate". A conjugate is typically a directly detectable label which has been attached or coupled to a specific binding member. "Specific binding member", as used herein, means a member of a binding pair, i.e., two different molecules where one of the molecules through, for example, chemical or physical means specifically binds to the other molecule. In addition to antigen and antibody specific binding pairs, other specific binding pairs include, but are not intended to be limited to, avidin and biotin; antibodies specific for haptens and haptens such as adamantane and carbazole which are described in U.S. Patent No. 5,424,414; complementary nucleotide sequences; enzyme cofactors or substrates and enzymes; and the like. Coupling chemistries for synthesizing a conjugate are well known in the art and can include, for example, any chemical means and/or physical means that does not destroy the specific binding property of the specific binding member or the detectable property of the label.

According to other detection embodiments, combinations of labels such as those described above, can be employed to detect hybrid dissociation. For example, a label, which is quenched or enhanced upon hybridization with a complementary sequence bearing a quenching or enhancing label, can be incorporated into the capture oligonucleotides or the target sequences. For instance, a label on single stranded capture oligonucleotide may emit a fluorescent signal, but when hybridized with a target sequence bearing a quenching or enhancing label, the signal may be quenched or the wavelength of the fluorescent signal may be different than when associated with the single stranded capture oligonucleotide exclusively. As a result, the presence of single stranded sequences or hybridized sequences can be detected.

Labels can be incorporated into repeat sequences or capture oligonucleotides by incorporating labels into amplification reagents or synthesis reagents such as, for example, nucleotides, primers or probes. Methods for labeling such reagents are well known in the art and have been described in, for example, U.S. Patent No. 4,948,882.

According to another embodiment of the invention, target sequences having a detectable label incorporated therein can be contacted with a repeat capture reagent comprising an array of two capture oligonucleotide sites on, for example, a glass chip. The first site may comprise oligonucleotides having a smaller multiple of the sequence complementary to the repeated sequence than the oligonucleotides at a second site. Upon formation of hybrids at the two capture oligonucleotide sites, a wash step optionally can be employed to separate unhybridized target sequences from the array, and a signal at both sites can be detected using methodologies previously described. Stringency may be increased with, for example, temperature increases and dissociation at the sites may be detected by observing signal loss at these sites. In the event the repeated sequence was present in the target sequence in a multiple less than the multiple of its complement in the capture oligonucleotides at either site, the signal generated at the sites would diminish at a common Tm. Detecting this dissociation pattern would indicate that the multiple of the repeated sequence was less than or equal to the multiple of its complement in the capture oligonucleotides at the first site. If, however, the repeated sequence was present in the target sequence in a multiple greater than the multiple of its complement in the capture oligonucleotide at the first site, but less than or equal to the multiple of its complement in the capture oligonucleotide at the second site, the target sequence would melt off first from the first capture site, followed by melting off the second capture site. Thus, the repeated sequence multiple in the repeat sequence could be determined (i.e. greater than the multiple of its complement at the first site) in a thermometer like fashion by observing a progressive loss of signal from the capture sites.

According to another embodiment, capture oligonucleotides can be randomly immobilized to a solid support material, at, for example, a single site. According to this embodiment, progressive dissociation events can be counted to determine the multiple of the repeated sequence in a target sequence. For example, three capture oligonucleotides having various multiples of the repeated sequences complement can be immobilized randomly to a single spot on a solid support material and contacted with a target sequence. After hybrid formation between the target sequence and various capture oligonucleotides, the stringency can be increased, and based upon the number of dissociation events detected, the multiple of repeated sequence in the target sequence can be determined. Thus, if all hybrids dissociated from the capture site at a common stringency, a single dissociation event would be detected and the multiple of the repeated sequence would be less than or equal to the multiple of its complement in the shortest capture oligonucleotide. Alternatively, if two dissociation events were detected, this would indicate that the target sequence dissociated from the shortest capture oligonucleotides first, followed by dissociation from the mid-sized and longest capture oligonucleotides; thus indicating that the repeated sequence multiple was greater than or equal to the multiple of its complement in the mid-sized oligonucleotide, but less than the number of its complement in the longest capture oligonucleotide. Similarly, if three dissociation events were detected, it could be determined that the repeated sequence multiple was greater than or equal to the multiple of its complement in the longest capture oligonucleotide.

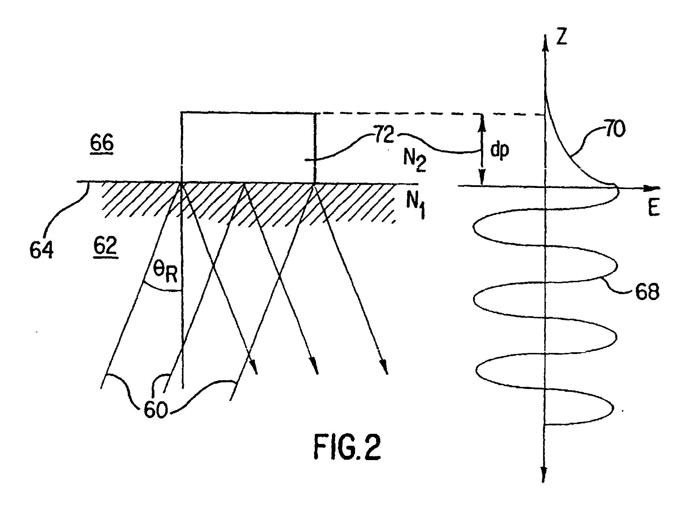

A preferred method for detecting a signal at arrayed capture oligonucleotide sites is found in WO-A-96 09532, which describes a detection method that employs an optical waveguide device to detect binding events, or the lack thereof, on a solid substrate. A waveguide device's ability to be employed in a hybridization type format is based upon a phenomenon called total internal reflection (TIR) which is known in the art. As illustrated in Figure 2, TIR operates upon the principle that light 60 traveling in a denser medium 62 (i.e. having the higher refractive index, $N_1$) and striking the interface 64 between the denser medium and a rarer medium 66 (i.e. having the lower refractive index, $N_2$) is totally reflected within the denser medium 62 if it strikes the interface at an angle, $\theta_R$, greater than the critical angle, $\theta_C$, where the critical angle is defined by the equation:

$$\theta_C = \arcsin (N_2/N_1).$$

Under these conditions, an electromagnetic waveform known as an "evanescent wave" is generated. As shown in Figure 2, the electric field associated with the light in the denser medium forms a standing sinusoidal wave 68 normal to the interface. The evanescent wave penetrates into the rarer medium 66, but its energy E dissipates exponentially as a function of distance Z from the interface as shown at 70. A parameter known as "penetration depth" ($d_p$- shown in Figure 2 at 72) is defined as the distance from the interface at which the evanescent wave energy has fallen to 0.368 times the energy value at the interface. [See, Sutherland et al., J. Immunol. Meth., **74**:253-265 (1984)] defining $d_p$ as the depth where $E= (e^{-1}) \cdot E_0$. Penetration depth is calculated as follows:

$$d_{\dot{p}} = \frac{\lambda/N_1}{2\pi\{\sin^2 q_R -(N_2/N_1)^2\}^{1/2}}$$

Factors that tend to increase the penetration depth are: increasing angle of incidence, $\theta_R$; closely matching indices of refraction of the two media (i.e. $N_2/N_1 \rightarrow 1$); and increasing wavelength, $\lambda$. For example, if a quartz TIR element ($N_1 = 1.46$) is placed in an aqueous medium ($N_2 = 1.34$), the critical angle, $\theta_C$, is 66° (= arcsin 0.9178). If 500 nm light impacts the interface at $\theta_R = 70°$ (i.e. greater than the critical angle) the $d_p$ is approximately 270 nm.

TIR has also been used in conjunction with light scattering detection in a technique referred to as Scattered Total Internal Reflectance ("STIR"). See, e.g., U.S. Patents 4,979,821 and 5,017,009 to Schutt, et al and WO 94/00763 (Akzo N. V.). According to this technique, a beam of light is scanned across the surface of a TIR element at a suitable angle and the light energy is totally reflected except for the evanescent wave. Particles such as red blood cells, colloidal gold or latex specifically bound within the penetration depth will scatter the light and the scattered light is detected by a photodetection means.

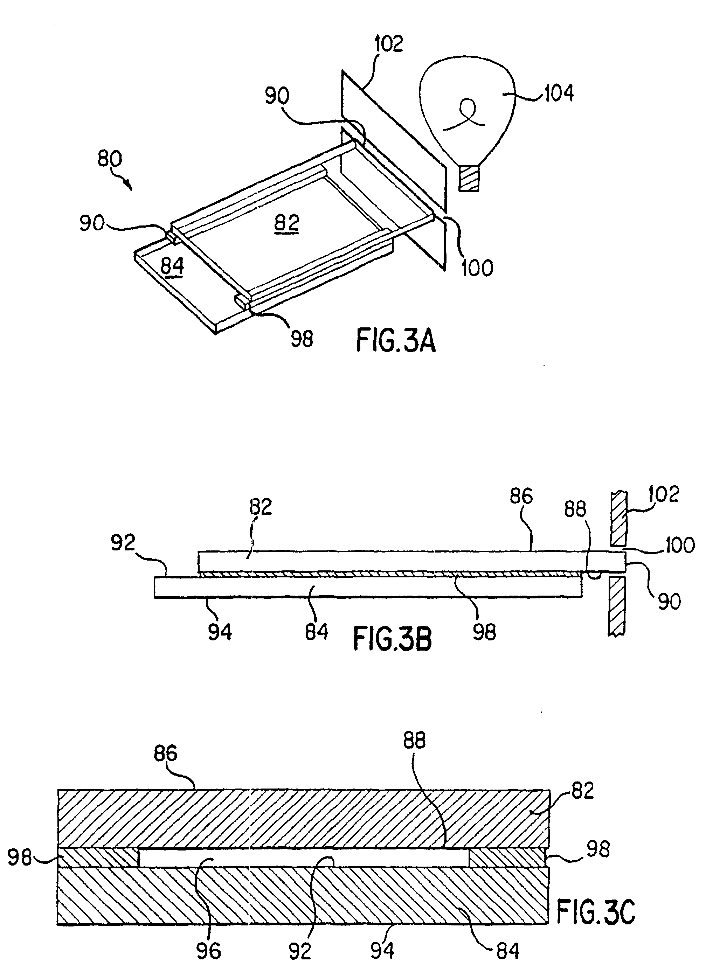

Figures 3A-3C illustrate a waveguide device 80 comprising a planar waveguide element 82 and a parallel planar plate 84. The waveguide element thus has parallel surfaces 86 and 88 as well as a light-receiving edge 90. Similarly, the plate 84 has parallel surfaces 92 and 94. The waveguide element 82 and the plate 84 are held together in spaced parallel fashion, such that the element surfaces 88 and the plate surface 92 define a narrow channel 96. The element and plate may be held together by any convenient means, including adhesive means 98 consisting of double stick tape disposed along the edges of the element and plate. The channel 96 is preferably rather small so as to enable capillary transfer of a fluid sample therethrough. For example, the height should be less than about 1mm, preferably less than about 0.1mm.

The element 82 should be made of an optically transparent material such as, for example, glass, quartz, or plastics such as polycarbonate, acrylic, or polystyrene. The refractive index of the waveguide must be greater than the refractive index of the sample fluid, as is known in the art for effecting total internal reflectance. For an aqueous sample solution, the refractive index, n, is about 1.33, so the waveguide typically has a refractive index of greater than 1.35, usually about 1.5 or more. The waveguide may be a piece of plastic or glass, for example, a standard glass microscope slide or cover slip may be used.

The plate 84 may be constructed of similar materials. As seen in Figures 3A and 3B, the light receiving end 90 of the waveguide element 82 is disposed in a narrow slit 100 of a mask 102 in order to minimize the effects of stray light originating from the light source 104. Minimization of stray light is also improved by the use of light absorbing materials.

Light source 104 for generating the incident light beam may be a source of electromagnetic energy, including energy in the visible, ultraviolet, and near-IR spectra. The term "light" is thus construed quite broadly and is not confined to the visible range, except in cases where detection is made visually. Non-visible wavelengths are detected by detectors optimized for the particular wavelength as is well known in the art. The light may be monochromatic or polychromatic, collimated or uncollimated, polarized or unpolarized. Preferred light sources include lasers, light emitting diodes, flash lamps, arc lamps, incandescent lamps and fluorescent discharge lamps. Thus, the light source used to illuminate the waveguide element can be a low wattage helium-neon laser or it may be a small incandescent light bulb powered by a battery, such as is used in pocket flashlight. Preferably, the light source includes potentiometer means for varying the intensity of the light source. Alternatively, filters and/or lenses may be employed to adjust the intensity to a suitable level.

Detection means may be employed to determine light scattering produced by a light scattering label (LSL). As seen best in Figure 3B, a LSL may be immobilized to surface 88 of waveguide element 82 via interactions between specific binding members such as, for example, that between an immobilized capture oligonucleotide and a cognate DNA sequence. A LSL is a molecule or a material, often a particle, which causes incident light to be scattered elastically, i.e. substantially without absorbing the light energy. Exemplary LSLs include colloidal metal and non-metal labels such as colloidal gold or selenium; red blood cells; and dyed plastic particles made of latex, polystyrene, polymethylacrylate, polycarbonate or similar materials. The size of such particulate labels ranges from 10 nm to 10 μm, typically from 50 to 500 nm, and preferably from 70 to 200 nm. The larger the particle, the greater the light scattering effect, and a unique feature of this system is the ability to quench scattering from the solution phase by the self-absorbancy of the particles. The upper size limit for the particles is not completely understood, but may be related to the increasing tendency of larger particles to self aggregate and precipitate. Suitable particle LSLs are available from Bangs Laboratories, Inc., Carmel, IN, USA.

Instrumentation and visual detection means may be employed to determine the degree of light scattering produced by a LSL. Light scattering events across the entire waveguicle can be monitored essentially simultaneously, whether by the naked eye of a human observer or by photodetection devices including CCD cameras which form images that are digitized and processed using computers.

The following examples are provided to further illustrate the present invention and not intended to limit the invention.

Example 1

Production of a Repeat Capture Reagent

In this example, repeat capture reagents designed to capture sequences having CTG and CGG repeats are produced.

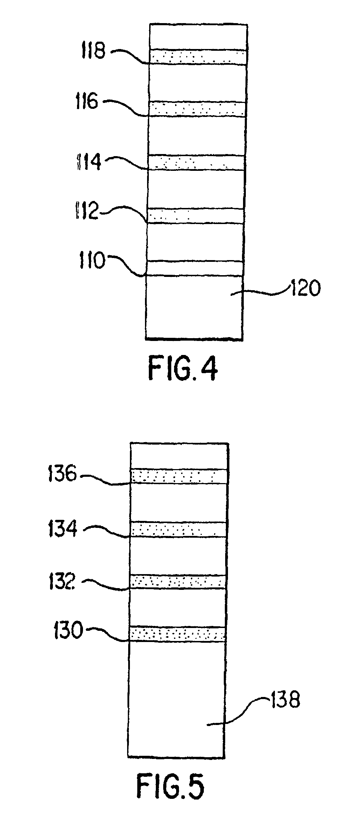

A schematic CTG repeat capture reagent is shown in Figure 4 where capture oligonucleotide sites 110, 112, 114, 116 and 118 are immobilized to a glass chip 120. Duplicate CTG capture reagents are produced where capture oligonucleotides comprising 10 (SEQ. ID. NO. 1), 20 (SEQ. ID. NO. 2), 30 (SEQ. ID. NO. 3), 40 (SEQ. ID. NO. 4) and 50 (SEQ. ID. NO. 5) repeats of the sequence GAC (i.e. CTG's complement) are immobilized to the chip in a manner represented in Figure 4, where band 110 illustrates the location of the 10 GAC repeat, band 112 illustrates the location of the 20 GAC repeat, band 114 illustrates the location of the 30 GAC repeat, band 116 illustrates the location of the 40 GAC repeat and band 118 illustrates the location of the 50 GAC repeat.

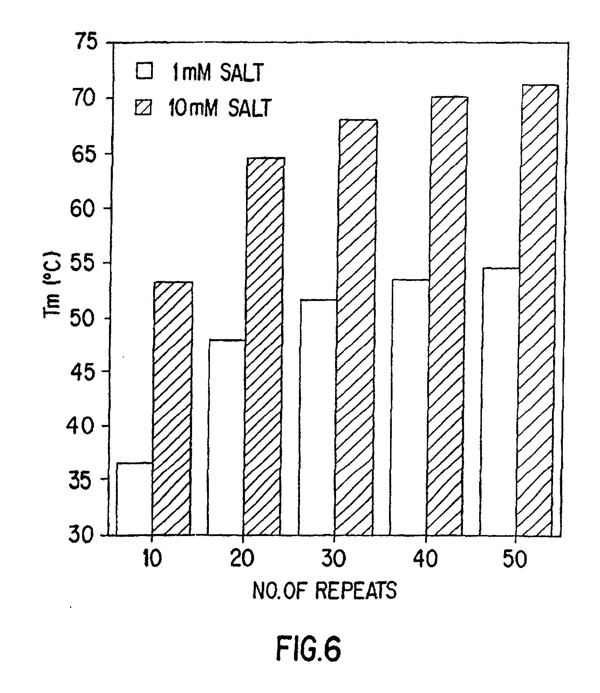

A schematic CGG repeat capture reagent is shown in Figure 5 where capture oligonucleotide sites 130, 132, 134 and 136 are immobilized to a glass chip 138. Duplicate CGG capture reagents are produced where capture oligonucleotides having 20 (SEQ. ID. NO. 6), 40 (SEQ. ID. NO. 7), 60 (SEQ. ID. NO. 8), and 80 (SEQ. ID. NO. 9) repeats of the GCC sequence (i.e. CGG's complement) are immobilized to the chip in a manner represented in Figure 5, where band 130 illustrates the location of the 20 GCC repeat, band 132 illustrates the location of the 40 GCC repeat, band 134 illustrates the location of the 60 GCC repeat and band 136 illustrates the location of the 80 GCC repeat.

Example 2

Length Determination of CTG Repeat Sequences

In this example nucleic acid sequences having 50 CTG repeats (SEQ. ID. NO. 10) are amplified and contacted with duplicate CTG repeat capture reagents produced in Example 1 and subject to incremental increases in temperature.

The CTG repeat sequences are amplified using 5' biotin labeled PCR primers that hybridize with the repeat region's flanking sequences. The sequences, and corresponding SEQ. ID. No.s, are found below in Table 2.

### Table 2

| 5'-Primer  Sequence-3' | SEQ. ID. No. |
|---|---|
| CACCACCAGCAACA | 11 |
| GGCCCAGACTTTCCGTGATG | 12 |

The samples are amplified by PCR for 30 cycles according to the method described in Sanpei, *et.al.*; *Biochemical Biophysical Research Communications,* Vol. 212, pp. 341-346 (July 17, 1995). The amplified samples are mixed with colloidal selenium conjugated with antibiotin as set forth in U.S. Patent Serial No. 08/311,462, filed September 22, 1994. Conjugated mixtures are then diluted down to defined salt concentrations of 10 mM or1 mM to affect the overall stringency and applied to the two CTG capture reagents under hybridizing conditions. After sufficient time for hybridization to occur between the hybridization oligonucleotides and the amplified target sequences has elapsed, unbound material is washed from the reagents with buffers having the defined salt concentrations, and, while in the buffer, the temperature in the environment of the hybrids is incrementally increased by raising the temperature in increments of 2°C and holding at each elevated temperature for approximately 1 minute. At each temperature increment, the presence or absence of the amplified sequences at the various capture sites is observed, by virtue of the label, to determine the dissociation pattern.

Figure 6 graphically illustrates the temperatures at which the various hybrids dissociate from the two capture reagents at the two salt concentrations. These melt temperatures were calculated using the software program "Oligo" version 4.03 which is available from National Biosciences; Plymouth, Minnesota. As shown by Figure 6, as the temperature increases the amplified sequences will progressively dissociate from the capture oligonucleotides with the capture oligonucleotide having 10 repeats dissociating first and the capture oligonucleotide having 50 repeats dissociating last for either salt concentration. However, the capture reagent having hybrids formed under higher salt conditions will consistently dissociate at higher temperatures than the hybrids formed under the lower salt conditions.

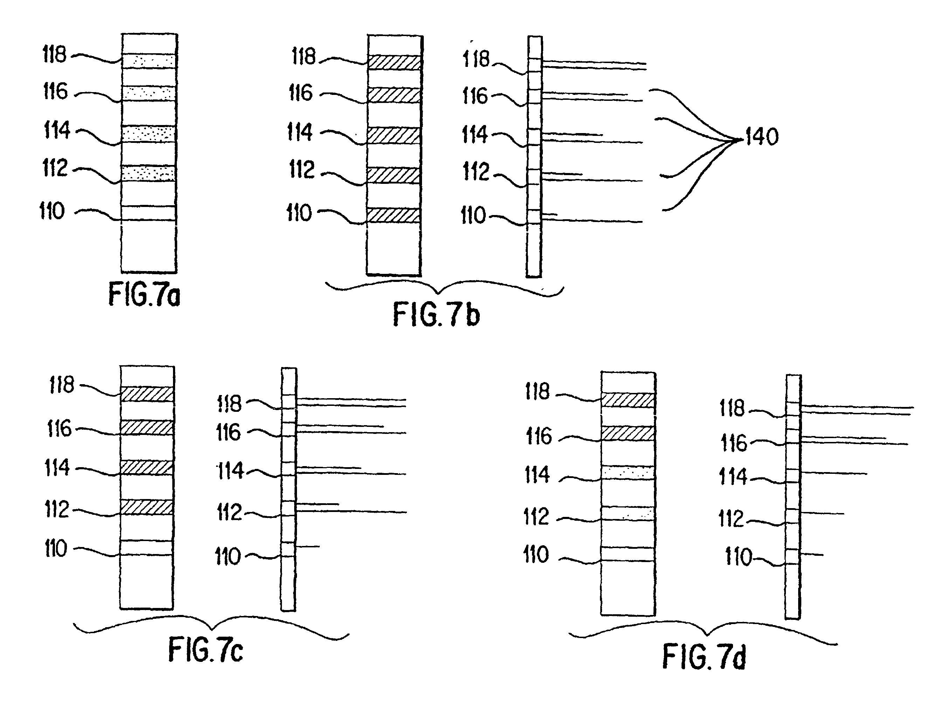

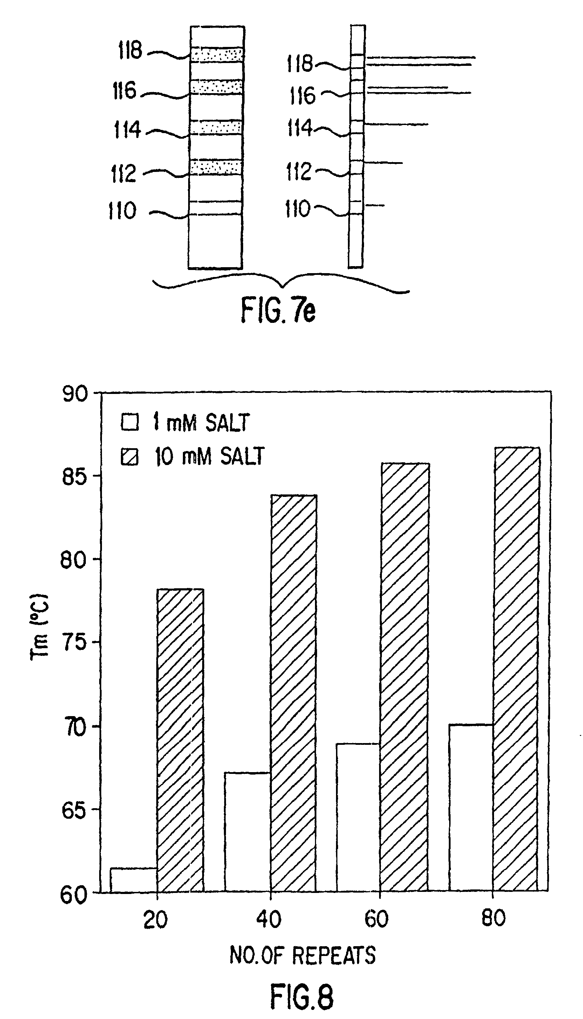

The progressive dissociation described in this example for the 10 mM salt concentration is shown schematically in Figures 7(a)-(e). Figure 7(a) is a top view of the GAC capture reagent as described in conjunction with Figure 4, prior to hybridization with the target sequences. Figure 7(b) shows a top and side view of the capture reagent after hybridization with the amplified target sequences. As shown in Figure 7(b), hybrids 140 are formed with all capture oligonucleotides with varying degrees of hybridization. As the temperature is increased to 60°C, dissociation occurs at band 110 where the amount of base pairing is the least and dissociation from band 110 is detected by the absence of hybridization at that band as illustrated in Figure 7(c). As the temperature is raised to approximately 68°C, hybrids at bands 112 and 114 are dissociated as shown in Figure 7(d). Finally, as the temperature is raised above 75°C, the final hybrids are released from the capture reagent as shown in Figure 7(e). Thus, the test sample contained a target sequence comprising a repeated sequence multiple of at least 50.

Example 3

Length Determination of CGG Repeat Sequences

In this example target sequences having 80 CGG repeats (SEQ. ID. NO. 13) are amplified as in Example 2, contacted with duplicate CGG repeat capture reagents as produced in Example 1 and subject to incremental increases in temperature.

The CGG repeat sequences are amplified using 5' biotin labeled PCR primers that hybridize with the repeat region's flanking sequences. The sequences, and corresponding SEQ. ID. No.s, are found below in Table 3.

### Table 3

| 5'-Primer  Sequence-3' | SEQ. ID. No. |
|---|---|
| ACCTCTGCAGAAATGGGCGTTCTGGCCCTC | 1 4 |
| GGCCCAGACTTTCCGTGATG | 1 5 |

The samples are amplified by PCR and the final reaction mixture contains 50mM KCl, 10mM Tris pH 8.3, 10mM MgCl$_2$, 10% DMSO, 200pM of each dNTP except 75% of dGTP was 7-deaza-dGTP (Pharmacia), 0 75 uM of each primer and 2-5 units of AmpliTaq enzyme (Perkin Elmer). The thermal cycling conditions used in the System 9600 Thermal Cycler (Perkin Elmer) were: 5 minutes initial melting at 95°C, 40 cycles of (95°C for 20 seconds, 60°C for 10 seconds, 72°C for 90 seconds), 5 minutes final extension at 72°C. The amplified samples are mixed with colloidal selenium conjugated with antibiotin as set forth in Example 2. Conjugated mixtures are then diluted down to defined salt concentrations of 10 mM or 1 mM to affect the overall stringency and applied to the two CGG capture reagents under hybridizing conditions. After sufficient time for hybridization to occur between the hybridization oligonucleotides and the amplified target sequences has elapsed, unbound material is washed from the reagents with buffers having the defined salt concentrations, and, while in the buffer, the temperature in the environment of the hybrids is incrementally increased by raising the temperature in increments of 2°C and holding at each elevated temperature for approximately 1 minute. At each temperature increment, the presence or absence of the amplified sequences at the various capture sites is observed, by virtue of the label, to determine the dissociation pattern.

Figure 8 graphically illustrates the temperatures at which the various hybrids dissociate from the two capture reagents at the two salt concentrations. The temperatures were created using the software program "Oligo" version 4.03 which is available from National Biosciences; Plymouth, Minnesota. As shown by Figure 8, as the temperature increases the amplified sequences will progressively melt or dissociate from capture oligonucleotides with the capture oligonucleotide having 20 repeats dissociating first and the capture oligonucleotide having 80 repeats dissociating last for either salt concentration. However, the capture reagent having hybrids formed under higher salt conditions will consistently dissociate at higher temperatures than the hybrids formed under the lower salt conditions.

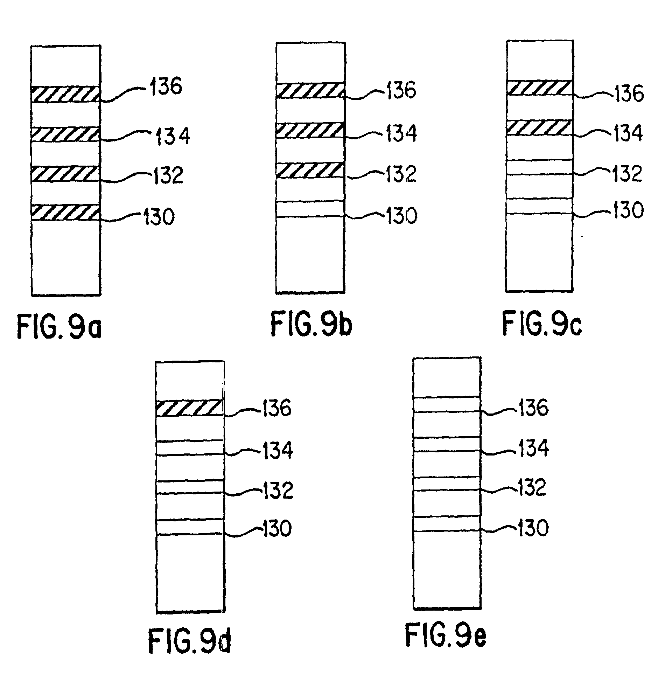

Figures 9(a)-9(e) schematically show the capture reagent signals as the temperature is increased, to thereby dissociate hybrids formed thereon, for the 10mM salt concentration. Hybridization at the sites is depicted as slanted lines. Figure 9(a) shows the CGG capture reagent, as shown in Figure 5, when hybrids are formed at all capture oligonucleotide sites 130, 132, 134, and 136. Figure 9(b) shows the capture reagent after the temperature is raised past approximately 77°C, the Tm of the capture oligonucleotides at site 130, after the hybrids at this site dissociate. Figure 9(c) shows the capture reagent after the temperature is raised past approximately 83°C, the Tm of the capture oligonucleotides at site 132, after the hybrids at this site dissociate. Figure 9(d) shows the capture reagent after the temperature is raised past approximately 85°C, the Tm of the capture oligonucleotides at site 134, after the hybrids at this site dissociate. Finally, Figure 9(e) shows the capture reagent after the temperature is raised past approximately 87°C, the Tm of the capture oligonucleotides at site 136, after the hybrids at this site dissociate. Based upon this progressive dissociation pattern, it is concluded that the multiple of the repeated CGG sequence in the repeat sequence was at least 80, the number of its complement found in the capture oligonucleotide at site 136.

### Example 4

Tri-Site Capture Reagent For Detection of Normal, Pre-Mutation and Full Mutation Triplet Sequences

In this Example a capture reagent designed to detect the relative length of a triplet repeat sequence using three capture sites is produced. The capture reagent is similar to the capture reagent shown in Figure 1. Capture oligonucleotides comprising 5 GAC repeats (SEQ. ID. NO. 16), 52 GAC repeats (SEQ. ID. NO. 17) and 100 GAC (SEQ. ID. NO. 18) repeats are immobilized, as shown in Figure 1, at sites 30, 40, and 50 respectively.

### Example 5

Detection of Normal, Pre-Mutation Myotonic Dystrophy and Full Mutation Myotonic Dystrophy Using a Tri-Site Capture Reagent

In This Example, samples from three patients are amplified using PCR, and the resulting amplicons are contacted with individual capture reagents synthesized in Example 4. The patient samples are from: a normal patient having between 5 and 37 CTG repeats; a pre-mutation individual having between 52 and 90 CTG repeats; and a full mutation individual having more than 100 CTG repeats.

The sequences are amplified using PCR primers labeled with the hapten adamantane. After each sample is amplified to generate multiple copies of the CTG containing target sequences, the amplified sequences are contacted with a selenium colloid conjugate comprising anti-adamantane antibody labeled with selenium colloid. After the conjugate is bound to the sequences, the labeled sequences are flowed into an optical waveguide device, as previously described and shown in Figure 3a, where the capture reagent of Example 4 comprises planar plate 84. The labeled sequences are allowed to hybridize to the various capture sites on the tri-site capture reagent, and the temperature is rapidly increased from approximately 28°C to approximately 115°C.

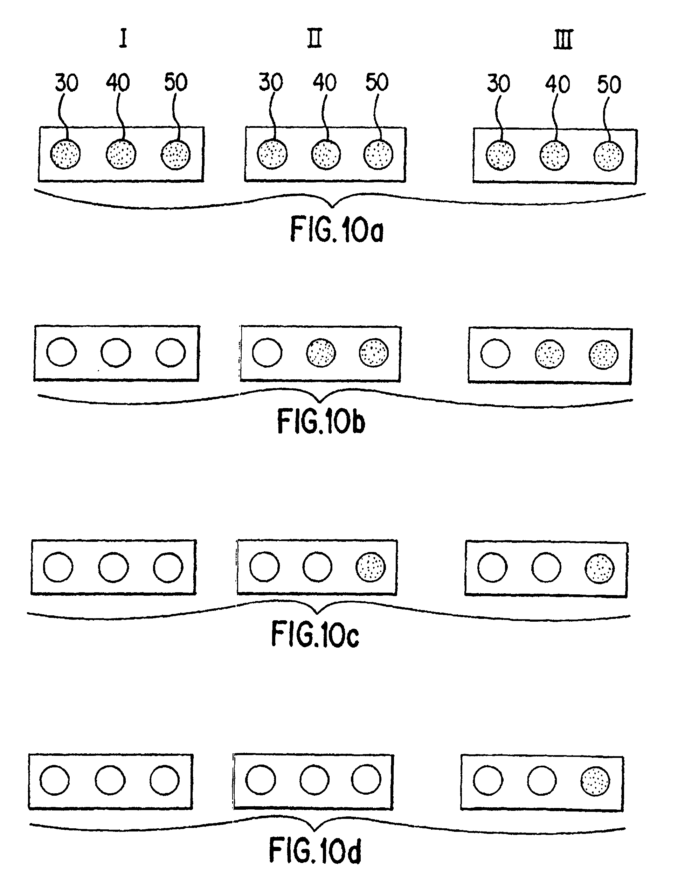

Figures 10(a)-10(d) represent the results of such an experiment where the capture reagents in row 10(a) represent the hybridization pattern at a first temperature (T1), the capture reagents in row 10(b) represent the hybridization pattern at a higher second temperature (T2), the capture reagents in row 10(c) represent the hybridization pattern at an even higher third temperature (T3), and the capture reagents in row 10(d) represent the hybridization pattern at a still higher fourth temperature (T4). The samples are identified above each column of tri-site capture reagents where Roman Numeral I represents sample from the normal patient, Roman Numeral II represents sample from the premutation patient, and Roman Numeral III represents sample from the full mutation patient. Darker shaded capture sites represent areas where amplified target sequences are bound.

As shown by Figures 10(a)-10(d), at T2 the capture sites for the normal patient are free of target sequences as they have dissociated from all capture sites because the repeated sequence multiple is not large enough to remain hybridized at the elevated temperatures T2-T4. While the sequences amplified from the pre-mutation patient are present at T1-T3, they are not long enough to remain hybridized when the temperature reaches T4. However, patient sample III, having a sequence of more than 100 CTG repeats, indicates hybridization at T4 because the repeated complementary region is sufficiently great to remain hybridized at T4. Thus, the progressive dissociation for sample III indicates that the repeated sequence multiple is greater than or equal to 100, the number of its complement in the capture oligonucleotide at site 50.

While the invention has been described in detail and with reference to specific embodiments, it will be apparent to one skilled in the art that various changes and modifications may be made to such embodiments without departing from the scope of the invention which is defined by the appended claims.

## Claims

1. A method of analyzing a target sequence comprising a repeated sequence, said repeated sequence having the formula $A_pC_yG_aT_z$ wherein T can be substituted with U; A, C, G, T and U represent bases; p, y, a, and z are between 0 and 5; the sum of p+y+a+z is at least 2; the repeated sequence is tandemly repeated at least twice in said target sequence; and A, C, G, and T or U can be in any order; said method comprising the steps of:

   a) contacting a test sample, comprising a target sequence, with a target sequence capture reagent to form target sequence/capture oligonucleotide hybrids wherein said target sequence capture reagent comprises at least a first and a second capture oligonucleotide immobilized to a solid support wherein

      (i) said first capture oligonucleotide comprises a first multiple of a sequence complementary to said repeated sequence,
      (ii) said second capture oligonucleotide comprises a second multiple of a sequence complementary to said repeated sequence, and
      (iii) said first multiple is different from said second multiple;

   b) increasing stringency in the environment of said hybrids to cause progressive dissociation of said hybrids;
   c) detecting said progressive hybrid dissociation; and

d) determining a multiple of said repeated sequence in said target sequence, wherein a determined multiple of said repeated sequence is relative to said capture oligonucleotides.

2. The method of claim 1 wherein detecting said progressive dissociation is achieved by monitoring a change in a detectable signal during said progressive hybrid dissociation.

3. The method of claim 1 wherein said first and said second capture oligonucleotide are immobilized to said solid support in an array.

4. The method of claim 1 further comprising a wash step, after said target sequence/capture oligonucleotide hybrids are formed and before increasing the stringency in the environment of said hybrids.

5. The method of claim 1 wherein said progressive dissociation is detected using an optical waveguide device.

6. The method of claim 1 further comprising a test sample pre-treatment step before contacting said test sample with said repeat capture reagent.

7. The method of claim 5 wherein said increasing the stringency in the environment of said hybrids is achieved by raising the temperature in the environment of said hybrids.

8. A target sequence capture reagent for capturing a target sequence comprising:

   a) at least two oligonucleotides wherein,

   (i) a first oligonucleotide comprises a first multiple of a sequence complementary to a repeated sequence,
   (ii) a second oligonucleotide comprises a second multiple of a sequence complementary to said repeated sequence, and
   (iii) said target sequence comprises said repeated sequence, said repeated sequence having the formula $A_pC_yG_aT_z$ wherein T can be substituted with U; A, C, G, T and U represent bases; p, y, a, and z are between 0 and 5; the sum of p+y+a+z is at least 2; the repeated sequence is tandemly repeated at least twice in said target sequence; and A, C, G, and T or U can be in any order; and

   b) a solid support material to which said at least two oligonucleotides are immobilized.

9. The capture reagent of claim 8 wherein said at least two oligonucleotides are immobilized to said solid support material in an array.

10. The repeat capture reagent of claim 8 wherein said capture reagent forms part of an optical waveguide device.

**Patentansprüche**

1. Ein Verfahren zur Analyse einer Targetsequenz, die eine wiederholte Sequenz umfasst, wobei die wiederholte Sequenz die Formel $A_pC_yG_aT_z$ hat, worin T durch U substituiert sein kann; A, C, G, T und U stellen Basen dar; p, y, a und z sind zwischen 0 und 5; die Summe von p+y+a+z ist mindestens 2; die wiederholte Sequenz wird direkt hintereinander liegend mindestens zweimal in der Targetsequenz wiederholt; und A, C, G und T oder U können in jeder beliebigen Reihenfolge sein; wobei das Verfahren die folgenden Schritte umfasst:

   a) In-Kontakt-bringen einer Testprobe, die eine Targetsequenz umfasst, mit einem Targetsequenzeinfangreagenz, um Targetsequenz/Einfang-Oligonucleotidhybride zu bilden, wobei das Targetsequenzeinfangreagenz mindestens ein erstes und ein zweites Einfangoligonucleotid umfasst, das an einem festen Träger immobilisiert ist, worin

   (i) das erste Einfangoligonucleotid ein erstes Vielfaches einer Sequenz umfasst, die komplementär zu der wiederholten Sequenz ist,
   (ii) das zweite Einfangoligonucleotid ein zweites Vielfaches einer Sequenz umfasst, die komplementär zu der wiederholten Sequenz ist, und
   (iii) das erste Vielfache sich von dem zweiten Vielfachen unterscheidet;

b) Erhöhen der Stringenz in der Umgebung der Hybride, um fortschreitende Dissoziation der Hybride zu veranlassen;

c) Detektieren der fortschreitenden Hybriddissoziation; und

d) Bestimmen eines Vielfachen der wiederholten Sequenz in der Targetsequenz, wobei ein bestimmtes Vielfaches der wiederholten Sequenz abhängig von den Einfangoligonucleotiden ist.

2. Das Verfahren von Anspruch 1, worin Detektieren der fortschreitenden Dissoziation erreicht wird durch Überwachen einer Änderung eines detektierbaren Signals während der fortschreitenden Hybriddissoziation.

3. Das Verfahren von Anspruch 1, worin das erste und das zweite Einfangoligonucleotid an dem festen Träger in einer Anordnung immobilisiert sind.

4. Das Verfahren von Anspruch 1, das weiter einen Waschschritt umfasst, nachdem die Targetsequenz/Einfangoligonucleotid-Hybride gebildet sind, und bevor die Stringenz in der Umgebung der Hybride erhöht wird.

5. Das Verfahren von Anspruch 1, worin die fortschreitende Dissoziation detektiert wird unter Verwendung einer optischen Wellenleitervorrichtung.

6. Das Verfahren von Anspruch 1, das weiter einen Testprobenvorbehandlungsschritt umfasst, bevor die Testprobe mit dem Wiederholungseinfangreagenz in Kontakt gebracht wird.

7. Das Verfahren von Anspruch 5, worin das Erhöhen der Stringenz in der Umgebung der Hybride erreicht wird, indem die Temperatur in der Umgebung der Hybride angehoben wird.

8. Ein Targetsequenzeinfangreagenz zum Einfangen einer Targetsequenz, das folgendes umfasst:

a) mindestens zwei Oligonucleotide, wobei

(i) ein erstes Oligonucleotid ein erstes Vielfaches einer Sequenz umfasst, die komplementär zu einer wiederholten Sequenz ist,

(ii) ein zweites Oligonucleotid ein zweites Vielfaches einer Sequenz umfasst, die komplementär zu der wiederholten Sequenz ist, und

(iii) die Targetsequenz die wiederholte Sequenz umfasst, wobei die wiederholte Sequenz die Formel $A_pC_yG_aT_z$ hat, worin T durch U substituiert sein kann; A, C, G, T und U stellen Basen dar; p, y, a und z sind zwischen 0 und 5; die Summe von p+y+a+z ist mindestens 2; die wiederholte Sequenz wird direkt hintereinander liegend mindestens zweimal in der Targetsequenz wiederholt; und A, C, G und T oder U können in jeder beliebigen Reihenfolge sein; und

b) ein festes Trägermaterial, an welchem die mindestens zwei Oligonucleotide immobilisiert sind.

9. Das Einfangreagenz von Anspruch 8, worin mindestens zwei Oligonucleotide an dem festen Trägermaterial in einer Anordnung immobilisiert sind.

10. Das Wiederholungseinfangreagenz von Anspruch 8, worin das Einfangreagenz einen Teil einer optischen Wellenleitervorrichtung bildet.

**Revendications**

1. Méthode d'analyse d'une séquence cible comprenant une séquence répétée, ladite séquence répétée ayant pour formule $A_pC_yG_aT_z$ où T peut être remplacé par U ; A, C, G, T et U représentent des bases ; p, y, a et z sont entre 0 et 5 ; la somme p+y+a+z est égale à au moins 2 ; la séquence répétée est répétée en tandem au moins deux fois dans ladite séquence cible ; et A, C, G et T ou U peuvent être dans un ordre quelconque ; ladite méthode comprenant les étapes consistant à :

(a) mettre en contact un échantillon à tester, comprenant une séquence cible, avec un réactif de capture de la séquence cible pour former des hybrides oligonucléotides de capture / séquence cible où ledit réactif de capture de la séquence cible comprend au moins un premier et un second oligonucléotide de capture immobilisés sur un support solide où

(i) ledit premier oligonucléotide de capture comprend un premier multiple d'une séquence complémentaire de ladite séquence répétée,
(ii) ledit second oligonucléotide de capture comprend un second multiple d'une séquence complémentaire de ladite séquence répétée et
(iii) ledit premier multiple est différent dudit second multiple ;

(b) augmenter la stringence dans l'environnement desdits hybrides pour entraîner la dissociation progressive desdits hybrides ;
(c) détecter ladite dissociation progressive des hybrides ; et
(d) déterminer un multiple de ladite séquence répétée dans ladite séquence cible, le multiple déterminé de ladite séquence répétée correspondant aux dits oligonucléotides de capture.

2. Méthode selon la revendication 1, dans laquelle la détection de ladite dissociation progressive est réalisée par le suivi d'une modification d'un signal détectable pendant ladite dissociation progressive des hybrides.

3. Méthode selon la revendication 1, dans laquelle lesdits premier et second oligonucléotides de capture sont immobilisés sur ledit support solide en rang.

4. Méthode selon la revendication 1, comprenant en outre une étape de lavage après la formation desdits hybrides séquence cible / oligonucléotides de capture et avant l'augmentation de la stringence dans l'environnement desdits hybrides.

5. Méthode selon la revendication 1, dans laquelle ladite dissociation progressive est détectée en utilisant un dispositif de guide d'ondes optique.

6. Méthode selon la revendication 1, comprenant en outre une étape de prétraitement de l'échantillon à tester avant la mise en contact dudit échantillon à tester avec ledit réactif de capture répété.

7. Méthode selon la revendication 5, dans laquelle ladite augmentation de la stringence dans l'environnement desdits hybrides est réalisée en augmentant la température dans l'environnement desdits hybrides.

8. Réactif de capture d'une séquence cible pour la capture d'une séquence cible comprenant :

(a) au moins deux oligonucléotides,

(i) un premier oligonucléotide comprenant un premier multiple d'une séquence complémentaire d'une séquence répétée,
(ii) un second oligonucléotide comprenant un second multiple d'une séquence complémentaire de ladite séquence répétée et
(iv) ladite séquence cible comprenant ladite séquence répétée, ladite séquence répétée ayant pour formule $A_pC_yG_aT_z$ où T peut être remplacé par U ; A, C, G, T et U représentent des bases ; p, y, a et z sont entre 0 et 5 ; la somme p+y+a+z est égale à au moins 2 ; la séquence répétée est répétée en tandem au moins deux fois dans ladite séquence cible ; et A, C, G et T ou U peuvent être dans un ordre quelconque ; et

(b) une matière support solide sur laquelle lesdits au moins deux oligonucléotides sont immobilisés.

9. Réactif de capture selon la revendication 8, dans lequel au moins deux oligonucléotides sont immobilisés sur ladite matière support solide en rang.

10. Réactif de capture répété selon la revendication 8, dans lequel ledit réactif de capture forme une partie d'un dispositif de guide d'ondes optique.

FIG.1

FIG.2

EP 0 876 511 B1

FIG.3A

FIG.3B

FIG.3C

118

116

114

112

110

120

FIG.4

136

134

132

130

138

FIG.5

FIG.6

FIG.7a

FIG.7b

FIG.7c

FIG.7d

FIG. 7e

FIG.8

FIG.9a

FIG.9b

FIG.9c

FIG.9d

FIG.9e

FIG.10a

FIG.10b

FIG.10c

FIG.10d